# EUROPÄISCHE PATENTANMELDUNG

(11) **EP 4 431 614 A1**
(43) Veröffentlichungstag der Anmeldung: **18.09.2024**
(21) Anmeldenummer: 23162092.3
(22) Anmeldetag: 15.03.2023
(51) Int. Cl.: C12P 19/02, C12N 9/04

(54) **VERFAHREN ZUR HERSTELLUNG VON WÄSSERIGEN LÖSUNGEN ENTHALTEND D-PSICOSE ODER L-PSICOSE**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend _{D}-Psicose oder _{L}-Psicose, indem aus D-Fructose, welche in einer wässerigen Lösung gelöst vorliegt, durch Behandeln mit einer Epimerase *in vitro* eine erste _{D}-Psicose gebildet wird, wonach die erste _{D}-Psicose durch Behandeln mit einer NAD(P)H-abhängigen Oxidoreduktase *in vitro* zu Allitol reduziert und nach Deaktivierung der Epimerase zur Bildung von _{D}-Psicose bzw. von _{L}-Psicose mit einer entsprechenden Oxidoreduktase behandelt wird, wonach die deaktivierte Epimerase und die Oxidoreduktasen entfernt werden. (Figur 1)

## Beschreibung

Die vorliegende Erfindung betrifft ein Verfahren zur Herstellung von wässerigen Lösungen der beiden seltenen Monosaccharide D-Psicose oder L-Psicose.

### Hintergrund der Erfindung

### D-Psicose

Das Monosaccharid D-Psicose, auch bekannt als D-Allulose, ist eine in der Natur selten vorkommende Ketohexose (Zhang et al., 2016). Sie konnte unter anderem in den Blättern von Rosmarinweiden (*Itea* sp.) nachgewiesen werden (Hough & Stacey, 1966), findet sich aber auch in verarbeiteten Lebensmitteln wie Konfekt und Gewürzsaucen, wo sie unter Einwirkung von Hitze aus D-Fructose, ihrem C₃-Epimer, entsteht (Oshima et al., 2006).

D-Psicose ist aufgrund ihres süßen Geschmacks für die Lebensmittelindustrie interessant. Im Vergleich zur Saccharose weist D-Psicose eine relative Süßkraft von 70% auf, besitzt aber einen niedrigen Energiegehalt (0,2 kcal/g), was einer Kalorien-Reduktion von etwa 95% (bezogen auf Saccharose) gleichkommt (Jiang et al., 2020).

In den USA ist D-Psicose von der US-amerikanischen Lebensmittelaufsicht Food and Drug Administration (FDA) als *Generally Recognized as Safe* (GRAS)-Lebensmittelzusatzstoff anerkannt, in der EU ist D-Psicose noch nicht als Lebensmittelzusatzstoff zugelassen (Ahmed et al., 2022).

Darüber hinaus hat D-Psicose auch positive Effekte auf den Lipid-Stoffwechsel sowie den Kohlenhydrat-Stoffwechsel (z.B. antidiabetisch) und wirkt entzündungshemmend und antioxidativ (Zhang et al., 2016; Jiang et al., 2020; Chen et al., 2022).

Aufgrund des geringen natürlichen Vorkommens wird D-Psicose weitgehend synthetisch (chemisch oder biotechnologisch) hergestellt.

Die Epimerisierung von D-Fructose zu D-Psicose kann durch Kochen in Pyridin unter Rückfluss mit anschließender Entfernung der anderen Hexosen durch Hefe-Fermentation durchgeführt werden, wobei allerdings nur 6,8 % der theoretischen Ausbeute an D-Psicose erreicht werden (Doner, 1979). Eine andere Methode beinhaltet die Epimerisierung von D-Fructose mit Molybdat-Ionen als Katalysator, wobei nur 0,5% der D-Fructose zu D-Psicose umgesetzt werden (Bilik & Tihlärik, 1974).

Die ineffizienten chemischen Syntheserouten wurden mittlerweile durch effizientere biotechnologische Verfahren ersetzt. Izumori et al. beschrieben 1993 eine Ketose-3-Epimerase aus *Pseudomonas cichorii* ST-24 zur Herstellung von D-Psicose aus D-Fructose (Izumori et al., 1993), welche auch patentiert wurde (EP 0592202 B1). Ketose-3-Epimerasen lassen sich je nach Substratspezifität in drei Gruppen einteilen: 1) D-Tagatose-3-Epimerase (DTE), 2) D-Psicose-3-Epimerase (DPE) oder D-Allulose-3-Epimerase (DAE) und 3) L-Ribulose-3-Epimerase (LRE) (Zhang et al., 2016; Jiang et al., 2020).

Die Umsetzung von D-Fructose zu D-Psicose mittels Ketose-3-Epimerasen läuft jedoch nicht vollständig ab, vielmehr bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose). Viele der Epimerasen benötigen außerdem ein zweiwertiges Metallion wie Mn2⁺ oder Co²⁺ (toxisch) als Kofaktor (Zhang et al., 2016; Jiang et al., 2020).

Das Gleichgewicht bei der Epimerisierung kann nicht nur durch Temperatur oder pH-Wert beeinflusst werden, sondern auch durch die Zugabe von (toxischem) Borat. Bedingt durch die bevorzugte Ausbildung eines D-Psicose-Borat-Komplexes wird das Gleichgewicht zur D-Psicose hin verschoben (Kim et al., 2008; Lim et al., 2009). EP 3643786 A2 und US 11028420 B2 beschreiben die chromatographische Auftrennung des D-Psicose-Borat-Komplexes mittels Simulated Moving Bed (SMB) Chromatographie. EP 3395952 B1 und US 10550414 B2 legen offen, dass die Konversion bei der Epimerisierung mittels DPE bei Zusatz von Natriumaluminat auf bis zu 67 % und bei Kaliumiodat auf bis zu 52% erhöht werden kann (Vergleich: 25% ohne Zusatz von Aluminat oder Iodat).

Zhu et al. (2020) präsentierten ein System bestehend aus zwei Enzymen (exo-Inulase aus *Bacillus velezenis* und DAE aus *Ruminococcus* sp.), mit dem Inulin aus *Helianthus tuberosus* L. (Topinambur) in ein Sirup bestehend aus D-Glucose, D-Fructose und D-Psicose (1:3:1) umgewandelt werden kann. Li et al. (2021a) verwendeten ein System bestehend aus Invertase, D-Glucose-Isomerase und immobilisierter DAE aus *Pirellula* sp. SH-Sr6A, um Saccharose, D-Glucose und D-Fructose (aus Fruchtsäften) in D-Psicose umzuwandeln. Es konnten 16 - 19% D-Psicose (bezogen auf den Gesamtgehalt der Kohlenhydrate) in den Säften angereichert werden.

Juneja et al. (2019) analysierten in einer Studie die technoökonomischen Aspekte eines modifizierten Corn-Dry-Grind-Prozesses, bei dem aus gemahlenem Mais zusätzlich zu Ethanol durch Einsatz eines modifizierten Hefestammes auch D-Psicose gebildet wird (durch Expression einer DPE). Die Autoren errechneten, dass aus einer Tonne Mais 390,4 l Ethanol und 75,3 kg D-Psicose gewonnen werden können und dass der minimale Verkaufspreis für die nach dem beschriebenen Verfahren erzeugte D-Psicose bei 1,29 US$/kg liegt (im Vergleich zum Marktpreis von 10 - 20 US$/kg im Jahr 2018). WO 2020/057560 A1 sowie WO 2020/057561 A1 beschreiben die Herstellung von D-Psicose aus Stärke mittels Saccharifizierung, enzymatischer Isomerisierung und Epimerisierung.

Patel et al. (2018) verwendeten eine auf magnetischen Eisenoxid-Nanopartikeln immobilisierte Smt3-DPE (Fusionsprotein) zur Herstellung von D-Psicose aus D-Fructose aus Obst-Trester-Waschlösungen. Die immobilisierte Epimerase konnte 20% der D-Fructose umsetzen und wurde nach Beendigung der Reaktion mit einem Magneten abgetrennt.

Yang et al. (2018) transferierten das DPE-Gen aus *Agrobacterium tumefaciens* in das thermotolerante Bakterium *Kluyveromyces marxianus.* So konnten in 12 h bei 55 °C aus 750 g/L D-Fructose 190 g/L D-Psicose hergestellt werden und die verbliebene D-Fructose wurde von *K. marxianus* zu Ethanol fermentiert. Dedania et al. (2020) immobilisierten DPE aus *Agrobacterium tumefaciens* auf Titandioxid-Nanopartikeln und konnten damit 36% der D-Fructose zu D-Psicose umsetzen. Darüber hinaus konnte das immobilisierte Enzym bis zu neunmal wiederverwendet werden.

Die bei der Epimerisierung von D-Fructose entstehenden D-Fructose/D-Psicose-Mischungen können entweder durch chromatographische Verfahren oder durch die "biologische Methode" (Fermentation der überschüssigen D-Fructose zu z.B. Ethanol) getrennt werden (Jiang et al., 2020). In US 20210189441 A1 wird die Trennung einer D-Fructose/D-Psicose-Mischung beschrieben, wobei die D-Fructose durch einen probiotischen Mikroorganismus (*Lactobacillus* oder *Saccharomyces*) in L-Milchsäure umgewandelt wird. EP 3423460 B1 beschreibt ein Verfahren zur Aufreinigung einer D-Fructose/D-Psicose-Mischung und zur Gewinnung hochreiner D-Psicose. EP 3553069 A1 legt eine Methode zur Trennung von D-Psicose und D-Fructose basierend auf SMB-Chromatographie offen.

Die Herstellung von D-Psicose über die Epimerase-Route hat allerdings folgende Nachteile: 1) Lage des Gleichgewichts aufseiten der D-Fructose, 2) Zusatz von (zum Teil toxischen) Metallionen als Kofaktoren für viele Epimerasen, 3) niedrige Aktivität und Langzeitstabilität der Epimerasen und 4) aufwendige Trennung des Produktgemisches.

Eine Möglichkeit zur Umgehung der thermodynamisch ungünstigen Epimerisierung sind Enzym-Kaskaden mit phosphorylierten Intermediaten. Der letzte Schritt, die Dephosphorylierung, ist irreversibel und treibt somit die Kaskade an (Li et al., 2021b).

Eine Kaskade, die in nahezu identischer Form sowohl von Li et al. (2021b) als auch von US 11168342 B2 und US 10907182 B2 beschrieben wird, weist D-Glucose-1-phosphat (G1P) als zentrales Intermediat auf. G1P wird mittels Phosphoglucomutase zuerst zu D-Glucose-6-phosphat (G6P) und dann weiter mittels Glucose-6-phosphat-Isomerase zu D-Frucose-6-phosphat (F6P) umgewandelt. F6P wird danach mittels D-Allulose-6-phosphat-Epimerase zu D-Psicose-6-phosphat epimerisiert, welches anschließend mit D-Allulose-6-phosphat-Phosphatase zur D-Psicose dephosphoryliert wird.

G1P kann beispielsweise durch die Einwirkung von Phosphorylasen auf z.B. Maltose und Amylodextrine (erhalten durch die Hydrolyse von Stärke), Cellodextrine (erhalten durch die Hydrolyse von Cellulose) oder Saccharose unter Verbrauch von Phosphat direkt hergestellt werden. Da die endständigen Zuckermonomere der Oligo- und Polysaccharide von den entsprechenden Phosphorylasen nicht phosphoryliert werden können, muss auf die Polyphosphat-Glucokinase (D-Glucose → G6P) oder Polyphosphat-Fructokinase (D-Fructose → F6P) zurückgegriffen werden, wobei noch zusätzlich Polyphosphate als Phosphat-Quelle zugesetzt werden müssen, um die Ausbeuten zu steigern (US 11168342 B2; US 10907182 B2). Als Substrat für die Kaskade von Li et al. (2021b) dient Stärke, welche zu D-Psicose mit Ausbeuten von 79% (bei 50 g/L Substrat-Konzentration; Reaktionszeit 24 h) umgewandelt wird.

Wang et al. entwickelten zur Herstellung von D-Psicose ebenfalls eine enzymatische Kaskade ausgehend von Stärke, welche allerdings über mehrere Schritte zu Glyceraldehyd-3-phosphat und Dihydroxyacetonphosphat umgesetzt wird. Dihydroxyacetonphosphat wird mit D-Glyceraldehyd unter Einwirkung von L-Fuculose-1-phosphat-Aldolase (FucA) zu D-Psicose-1-phosphat umgewandelt, welches mittels Phosphatase zu D-Psicose dephosphoryliert wird (95 % Ausbeute bei einem Titer von 15,2 mM). Glyceraldehyd-3-phosphat wird weiter zu 2-Deoxy-D-ribose umgesetzt (Wang et al., 2020).

Auch eine Enzymkaskade ausgehend von Glycerin ist in der Literatur beschrieben. Dieses wird zu Dihydroxyacetonphosphat (Phosphorylierung von Glycerin mit einer sauren Phosphatase und nachfolgende Oxidation mit einer Glycerinphosphat-Oxidase) und D-Glyceraldehyd (Oxidation von Glycerin mit Alditol-Oxidase) umgesetzt, welche wiederum als Substrat für eine Aldolase (wie z.B. FucA) dienen. Nach Abspaltung der Phosphatgruppe wird eine Mischung aus D-Sorbose und D-Psicose erhalten, die chromatographisch getrennt werden kann (Li et al., 2020). WO 2016/201110 A1 wiederum beschreibt eine Methode zur Herstellung von D-Psicose aus Dihydroxyaceton und D-Glyceraldehyd mittels Fructose-6-phosphat-Aldolase und DTE (Zwischenprodukt D-Fructose).

Xiao et al. koppelten die Epimerisierung von D-Fructose mit der Umwandlung der D-Psicose zu D-Psicose-1-phosphat mittels L-Rhamnulose-Kinase (unter Verbrauch von Adenosintriphosphat (ATP)), um das Gleichgewicht der Epimerisierung zu verschieben. Durch nachfolgende Abspaltung der Phosphat-Gruppe durch eine saure Phosphatase wird D-Psicose erhalten (99% Umsatz von 20 mM D-Fructose). ATP muss allerdings unter Zusatz von Polyphosphat mit einer Polyphosphat-Kinase regeneriert werden (Xiao et al., 2019).

Ein großer Nachteil der Routen mit phosphorylierten Intermediaten ist der Einsatz von teuren, energiereichen Phosphat-Verbindungen wie Polyphosphat oder ATP in stöchiometrischen Mengen zur Einführung der Phosphat-Gruppen. Durch die Verwendung von Phosphorylasen kann dieses Problem zum Teil umgangen werden, jedoch können endständige Monosaccharide nicht ohne Zuhilfenahme von energiereichen Phosphat-Verbindungen phosphoryliert werden. Außerdem müssen die verbliebenen Phosphat-Verbindungen und Phosphat-Ionen nach Beendigung der Reaktion entfernt werden.

Auch Fermentationsprozesse zur Herstellung von D-Psicose sind bekannt. Zhang et al. (2021) präsentierten einen Fermentationsprozess basierend auf der Kokultivierung von engineerten *Bacillus subtilis* und *Escherichia coli* zur gemeinsamen Produktion von D-Psicose (Titer 11,7 g/l; Umsatz: 69,5%) und dem Enzym Lipase. In US 2017/0298400 A1 wird die Expression von DPE (z.B. aus *Agrobacterium tumefaciens*) in verschiedenen Mikroorganismen beschrieben. EP 3088515 B1 und US 9701953 B2 beschreiben einen D-Psicose produzierenden *Ensifer adhaerens*-Stamm. In EP 2470668 B1 wird die Immobilisierung eines GRAS-Mikroorganismus (*Corynebacterium glutamicum* KCCM 11046) mit exprimierter DPE auf einem Natriumalginat-Träger offengelegt.

### L-Psicose

Im Vergleich zu ihrem Enantiomer D-Psicose kommt L-Psicose natürlich nicht vor. L-Psicose dient als Ausgangsmaterial zur Herstellung wie L-Fructose (Itoh & Izumori, 1996), L-Tagatose (Rao et al., 2008) oder L-Talitol (Sasahara & Izumori, 2005). In einer Studie mit Mäusen konnte die antivirale Aktivität von L-Psicose gegenüber dem Herpes-simplex-Virus 1, das eine Keratitis (Hornhautentzündung) auslöst, festgestellt werden (Muniruzzaman et al., 2016).

Zur Herstellung von L-Psicose sind im Wesentlichen drei verschiedene biotechnologische Routen bekannt.

Durch die Aldol-Reaktion von L-Glyceraldehyd und Dihydroxyacetonphosphat (DHAP), katalysiert durch eine Fructose-1,6-diphosphat-Aldolase oder eine Tagatose-1,6-diphosphat-Aldolase, mit anschließender Abspaltung der endständigen Phosphat-Gruppe wird eine Mischung aus L-Psicose und L-Sorbose erhalten. Die Reaktionskaskade kann sowohl *in vitro* als auch fermentativ in einem engineerten *Corynebacterium glutamicum*-Stamm durchgeführt werden (Yang et al., 2015). Yang et al. (2016) entwickelten die Reaktionskaskade weiter, sodass auch Glycerin als Startmaterial für die fermentative Herstellung von L-Psicose verwendet werden kann. Das Herzstück der Kaskade ist ebenfalls die Aldol-Reaktion von L-Glyceraldehyd und DHAP.

Ebenfalls von L-Glyceraldehyd und Dihydroxyacetonphosphat (DHAP) startet die Route von Wen et al. (2015), die zuerst mit einer Rhamnulose-bisphosphat-Aldolase zu L-Fructose-1-phosphat umgesetzt werden, aus der nach Dephosphorylierung L-Fructose entsteht. Diese wird mit einer DTE zu L-Psicose isomerisiert, wobei das Gleichgewicht durch Phosphorylierung mittels Fructokinase (benötigt Adenosintriphosphat (ATP) als Kofaktor) zur Produktseite verschoben wird. Nach Entfernung der Phosphat-Gruppe wird L-Psicose erhalten.

Eine Alternative zur Aldol-Reaktion ist die Verwendung von Transketolase (EC 2.2.1.1), einem Thiaminabhängigen Enzym, das die Reaktion zwischen einem Donor mit einer α-Hydroxy-Carbonyl-Gruppe und einem Cₙ-Aldehyd-Akzeptor katalysiert, wobei unter CO₂-Abspaltung eine Cₙ₊₂-Ketose gebildet wird. So kann L-Psicose durch Reaktion von L-Erythrose mit Hydroxypyruvat mittels einer thermostabilen Transketolase-Mutante aus *Geobacillus stearothermophilus* hergestellt werden, allerdings wird als Kofaktor Thiamindiphosphat benötigt (Lorillière et al., 2019).

### Allitol als Intermediat

Die unvorteilhafte Lage des Gleichgewichts der Epimerisierung von D-Fructose zu D-Psicose kann auch durch nachgeschaltete Redoxreaktionen günstig beeinflusst werden. Durch Kombination einer DTE mit einer Ribitol-Dehydrogenase und Formiat-Dehydrogenase (als Regenerationsenzym für den Kofaktor Nicotinamidadenindinukleotid (NAD)) kann D-Fructose zum Zuckeralkohol Allitol umgewandelt werden (Takeshita et al., 2000).

Durch einen Oxidationsschritt kann Allitol im Anschluss wieder zu D-Psicose umgesetzt werden. Dies kann beispielsweise mikrobiell mit *Enterobacter aerogenes* IK7 (vollständige Oxidation von 100 g/l Allitol in 24 h) oder *Bacillus pallidus* Y25 (48% Umsatz von 50 g/l Allitol in 48 h) bewerkstelligt werden (Gullapalli et al., 2007; Poonperm et al., 2007).

Der achirale Zuckeralkohol Allitol bildet aufgrund seiner Symmetrie eine Schnittstelle zwischen den D- und L-Hexosen in der sogenannten Izumoring-Strategie zur Bioproduktion von seltenen Zuckern (Izumori, 2006; Hassanin et al., 2017). Somit kann er auch als Vorstufe zur Herstellung von weiteren seltenen Monosacchariden dienen.

Ebenfalls durch mikrobielle Oxidation kann Allitol zu L-Psicose umgewandelt werden - mit *Gluconobacter frateurii* IFO 3254 können 100 g/l Allitol zu 98% zu L-Psicose oxidiert werden (Takeshita et al., 1996). Die Oxidation durch denselben Organismus ist auch in JP4761424B2 sowie JP3711296B2 beschrieben.

Die genannten Verfahren zur Herstellung wässeriger Lösungen von D-Psicose oder L-Psicose haben den Nachteil, dass entweder die Ausbeuten gering sind oder dass Reaktionsbedingungen und abschließende Reinigungsschritte angewendet werden müssen, die einer wirtschaftlichen Verwertung entgegenstehen.

Hier setzt nun die Aufgabe der vorliegenden Erfindung an. Die Erfindung möchte ein effizientes und kostengünstiges Verfahren zur Herstellung von wässerigen Lösungen enthaltend D-Psicose oder L-Psicose mit hohem Umsatz zur Verfügung zu stellen, welches vorzugsweise im Eintopf-Verfahren durchgeführt werden kann, und bei welchem eine chromatographische Reinigung nicht vorgenommen zu werden braucht.

### Detaillierte Beschreibung der Erfindung

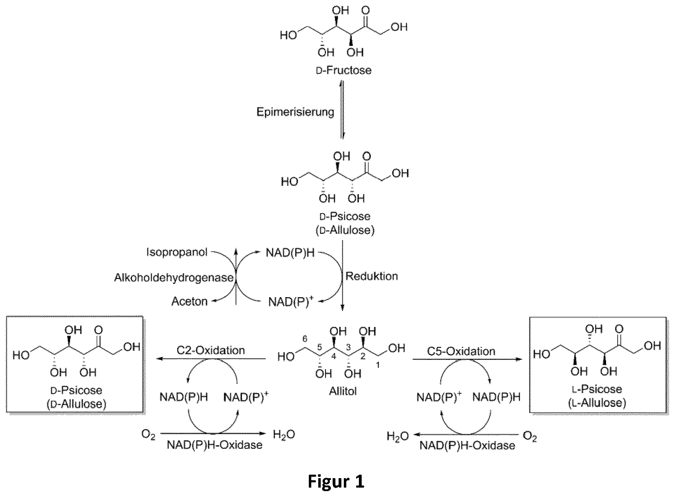

Die Aufgabe wird erfindungsgemäß gelöst, indem aus D-Fructose, welche in einer wässerigen Lösung gelöst vorliegt, durch Behandeln mit einer Epimerase *in vitro* eine erste D-Psicose gebildet wird, wonach die erste D-Psicose durch Behandeln mit einer NAD(P)H-abhängigen Oxidoreduktase *in vitro* zu Allitol reduziert und nach Deaktivierung der Epimerase zur Bildung von D-Psicose bzw. von L-Psicose mit einer entsprechenden Oxidoreduktase behandelt wird, wonach die deaktivierte Epimerase und die Oxidoreduktasen entfernt werden. Das erfindungsgemäße Verfahren ist in Figur 1 schematisch dargestellt.

Es hat sich überaschenderweise gezeigt, dass die erfindungsgemäß gesetzten Ziele erreicht werden können, wenn die Umsetzung nicht fermentativ durchgeführt wird, sondern die Enzyme als solche in der wässerigen Lösung enthalten sind, also *in vitro* gearbeitet wird.

Eine bevorzugte Variante des erfindungsgemäßen Verfahrens besteht darin, dass der durch die Reduktion entstandene oxidierte Kofaktor NAD(P)⁺ mittels einer Alkoholdehydrogenase und einem sekundären Alkohol unter Bildung eines Ketons reduziert wird, wobei der sekundäre Alkohol 2-bevorzugt Propanol ist.

Eine weitere bevorzugte Variante des erfindungsgemäßen Verfahrens ist dadurch gekennzeichnet, dass es als Eintopf-Reaktion ohne Isolierung von Zwischenprodukten durchgeführt wird.

Im erfindungsgemäßen Verfahren werden die Enzyme vorzugsweise als Lysat der entsprechenden, sie bildenden Zellen eingesetzt.

Vor der Ausführung des letzten Schritts (Oxidation) werden die Enzyme (Epimerase und Reduktase/Dehydrogenase) des ersten Schritts (D-Fructose → Allitol) durch Hitzeeinwirkung deaktiviert oder durch Ultrafiltration entfernt, um die Bildung von Nebenprodukten durch die Enzyme zu verhindern. Besonders im Fall von D-Psicose würde ohne die entsprechende Behandlung ein Großteil der durch die Oxidation entstandenen D-Psicose von der Epimerase wieder zu D-Fructose umgewandelt werden.

Die Regeneration der Nicotinamid-basierenden Kofaktoren (NAD oder NADP) erfolgt im Falle der Reduktion der D-Psicose zu Allitol bevorzugt mit einer NAD(P)-abhängigen Alkoholdehydrogenase und im Falle der Oxidationsreaktionen (zweiter Schritt) mit einer H₂O-bildenden NAD(P)H-Oxidase.

Als Substrat für die zur Kofaktor-Regeneration bei der Reduktion eingesetzte Alkoholdehydrogenase wird ein Alkohol, bevorzugt Isopropanol (2-Propanol), verwendet.

Gemäß einer bevorzugten Ausführungsform des erfindungsgemäßen Verfahrens wird D-Fructose als Substrat eingesetzt.

Die besonders bevorzugte Konzentration von D-Fructose beträgt 50 - 250 g/l.

Der besonders bevorzugte Temperaturbereich für den ersten Schritt (Epimerisierung und Reduktion) liegt zwischen 25 und 45 °C, für den zweiten Schritt (Oxidation) zwischen 24 und 30 °C.

Der besonders bevorzugte pH-Bereich beider Schritte liegt zwischen 7 und 8,5.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund des nicht Vorhandensein von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme und Herstellung der Lysate* für Details).

In einer weiteren Variante können die Enzyme auch auf einem festen Trägermaterial immobilisiert sein.

In einer besonders bevorzugten Ausführung des Verfahrens werden für die Konversion des Startmaterials nur Enzyme aus den Enzymgruppen Epimerasen, Dehydrogenasen, Reduktasen und Oxidasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Das zur Reduktion von D-Psicose verwendete Enzym kann aus der Gruppe der Oxidoreduktasen stammen, wobei eine Short-Chain-Dehydrogenase/Reduktase (SDR) besonders bevorzugt ist.

Das zur Oxidation von Allitol zu D-Psicose verwendete Enzym kann aus der Gruppe der Oxidoreduktasen stammen, wobei eine Xylitol-Dehydrogenase (EC 1.1.1.9) oder eine Short-Chain-Dehydrogenase/Reduktase (SDR) besonders bevorzugt sind.

Das zur Oxidation von Allitol zu L-Psicose verwendete Enzym kann aus der Gruppe der Oxidoreduktasen stammen, wobei eine Mannitol-Dehydrogenase-artige Short-Chain-Dehydrogenase/Reduktase besonders bevorzugt ist.

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Die hier vorgestellte enzymatische Strategie minimiert die Anzahl der Enzyme und erlaubt somit einen hocheffizienten und kostengünstigen/preislich kompetitiven Bioproduktionsprozess.

### Materialien

D-Psicose wurde von TCI und Hunan Garden Naturals Inc. (China), Allitol und L-Psicose wurden von TCI, D-Fructose, Lysozym, NADPH-Tetranatriumsalz, und Methanol wurden von PanReac AppliChem (ITW Reagents), Zinkchlorid, IPTG (Isopropyl-β-D-thiogalactopyranosid) wurden von Sigma-Aldrich, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien werden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert. Die rekombinante Expression des Target-Enzymes erreicht meistens 50 % des *E. coli*-Gesamtproteins.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Die Homogenate wurden 10 min lang bei 4 °C und 16000 rpm zentrifugiert (Hermle Z 446 K Zentrifuge), um die unlöslichen Zellfragmente abzutrennen und das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Spenderorganismus** | **Aufschlussbedingungen** | **Literatur** |
|---|---|---|---|---|
| D-Psicose-3-Epimerase (EC 5.1.3.30) | D-Fructose → D-Psicose | *Clostridium cellulolyticum* H10 | GEA (20% Biomasse in 100 mM TEA-HCl pH 7 + 2 mM MgCl₂) | (Mu et al., 2011; Chan et al., 2012) |
| Short-Chain-Dehydrogenase /Reduktase (SDR) | D-Psicose → Allitol (+ Rückreaktion) | *Gluconobacter frateurii* (DSM 7146) | GEA (20% Biomasse in 100 mM TEA-HCl pH 7) | (NLM Protein database: WP_063903495.1) |
| Xylitol-Dehydrogenase (XDH) (EC 1.1.1.9) | Allitol → D-Psicose | *Galactocandida mastotermitis* (*Candida* sp. HA167) | | (Habenicht et al., 1999) |
| Mannitol-Dehydrogenase-artige Short-Chain-Dehydrogenase /Reduktase | Allitol → L-Psicose | *Millerozyma farinosa (Pichia sorbitophila)* CBS 7064 | GEA (20% Biomasse in 100 mM TEA-HCl pH 7) | (Louis et al., 2012) |
| Alkoholdehydro genase (ADH) (EC 1.1.1.1) | Isopropanol → Aceton | *(Geo-)Bacillus stearothermophilus* NCA1503 | GEA (20% Biomasse in 100 mM TEA-HCl pH 7 + 0,5 mM ZnCl₂) | (Sakoda & Imanaka, 1992) |
| NADH-Oxidase (EC 1.6.3.4) | NADH → NAD⁺ | z. B. *Streptococcus mutans* | GEA (20% Biomasse in 100 mM KPP pH 7) | (Matsumoto et al., 1996) |
| NAD(P)H-Oxidase (EC 1.6.3.2) | NAD(P)H → NAD(P)⁺ | z*.*B*. Streptococcus mutans*; Mutante | | (Petschacher et al., 2014) |

Die Verwendung der in der obigen Tabelle angegebenen Enzyme Short-Chain-Dehydrogenase/Reduktase (SDR), Xylitol-Dehydrogenase (XDH, EC 1.1.1.9) und Mannitol-Dehydrogenase-artige Short-Chain-Dehydrogenase/Reduktase für die ebenfalls in der Tabelle angegebenen katalysierten Reaktionen ist nicht bekannt und ihre Eignung war auch nicht zu erwarten. Ein weiterer Aspekt der vorliegenden Erfindung betrifft daher auch die in der Tabelle angegebene Verwendung dieser Enzyme.

### Analytische Methoden

### High Performance Liquid Chromatography

Zur Quantifizierung von D-Psicose, L-Psicose, D-Fructose sowie der Zwischenstufe Allitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Umsatz von D-Fructose zu D-Psicose (Eintopf-Verfahren)

Die Reaktion wurde in einem Labfors 5 Tisch-Bioreaktor (Infors AG) durchgeführt. Als Gefäß wurde ein Glasreaktor (Volumen 3,4 l) mit aufgesetztem Rührwerk, pH-Elektrode und O₂-Sensor verwendet. Die pH-Kontrolle erfolgte durch die Zudosierung von 1M NaOH oder 1M H₂SO₄.

Zu Beginn wurden 50 ml einer D-Fructose-Lösung (500 g/l), 246,1 ml deionisiertes Wasser sowie 96 ml eines 200 mM TEA-HCl-Puffers (pH 8) im Reaktor vorgelegt und unter Rühren auf 35 °C gebracht.

Zum Start der Reaktion wurden 25 ml D-Psicose-3-Epimerase-Lysat zugesetzt. Danach wurden 25 ml Short-Chain-Dehydrogenase/Reduktase-Lysat, 4 kU Alkoholdehydrogenase-Lysat, 10 ml einer 10 mM NAD⁺-Lösung sowie 40 ml Isopropanol eingebracht.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (Rl-Detektion) vermessen.

Nach 23 h Laufzeit wurden 15 ml, nach 50 h wurden 20 ml und nach 77 h wurden 15 ml Isopropanol nachdosiert. Nach 74 h wurden 5 ml D-Psicose-3-Epimerase-Lysat, nach 77 h wurden 15 ml Short-Chain-Dehydrogenase/Reduktase-Lysat und 2,4 kU Alkoholdehydrogenase-Lysat zugegeben.

Nach 97 h wurden 94% der D-Fructose (50 g/l) zu Allitol umgesetzt (gefundene Konzentration: 42 g/l).

Danach wurde der gesamte Reaktorinhalt für 60 min auf 70 °C erhitzt (Deaktivierung der D-Psicose-3-Epimerase) und nach Abkühlen auf 24 °C wurden 25 ml Xylitol-Dehydrogenase-Lysat, 10 kU NADH-Oxidase-Lysat sowie 10 ml einer 10 mM NAD⁺-Lösung zugesetzt.

Allitol wurde innerhalb von 3 h vollständig zu D-Psicose oxidiert. Der Reaktorinhalt wurde auf 70 °C erhitzt, der pH-Wert auf 4 eingestellt und für 30 min bei 70 °C gerührt. Die Enzyme wurden über eine Glasfritte (P3) abfiltriert.

Im Filtrat wurden 34,7 g/l D-Psicose detektiert.

Das Filtrat wurde bis zu einer D-Psicose-Konzentration von 520 g/l am Rotationsverdampfer aufkonzentriert.

Das Beispiel 1 zeigt, dass die Epimerase durch Hitzeeinwirkung (im Eintopf-Verfahren) denaturiert werden kann und der entstandene Niederschlag die weitere Reaktionsführung nicht stört.

### Beispiel 2

### Umsatz von D-Fructose zu D- und L-Psicose (Eintopf-Verfahren)

In zwei 2 ml Glas-Vials (Vial 1, Vial 2) wurden folgende Komponenten vermischt: 70,6 µl deionisiertes Wasser, 250 µl eines 200 mM TEA-HCl-Puffers (pH 8), 50 µl einer D-Fructose-Lösung (500 g/l) und 25 µl D-Psicose-3-Epimerase-Lysat. Anschließend wurden 35 µl Short-Chain-Dehydrogenase/Reduktase-Lysat, 8 U Alkoholdehydrogenase-Lysat, 5 µl einer 10 mM NAD⁺-Lösung sowie 50 µl Isopropanol zu beiden Ansätzen hinzugefügt. Die Ansätze wurden unter kontinuierlichem Schütteln (Eppendorf-Thermomixer; 35 °C, 800 rpm) für insgesamt 20 h inkubiert.

Beide Ansätze wurden 60 min auf 70 °C erhitzt. Nach Abkühlen wurden 25 µl Oxidoreduktase-Lysat (Vial 1: Mannitol-Dehydrogenase-artige Short-Chain-Dehydrogenase/Reduktase; Vial 2: Short-Chain-Dehydrogenase/Reduktase), 10 U NAD(P)H-Oxidase-Lysat sowie 5 µl einer 5 mM NADP⁺-Lösung zu beiden Ansätzen zugesetzt. Die Ansätze wurden in einem Eppendorf-Thermomixer unter kontinuierlichem Schütteln (24 °C, 800 rpm) für weitere 20 h inkubiert.

Zur Analyse wurden 100 µl des Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden in Vial 1 97,7% der D-Fructose zu L-Psicose (gefundene Menge: 45,8 g/l) und in Vial 2 68,7% der D-Fructose zu D-Psicose umgesetzt (gefundene Menge: 33,2 g/l).

D-Psicose und L-Psicose können von der verwendeten chromatographischen Methode nicht unterschieden werden (Elution bei derselben Retentionszeit), daher wurden 25 µl D-Psicose-3-Epimerase-Lysat zu beiden Vials zugesetzt. Die Ansätze wurden für 2 h bei 35 °C inkubiert und erneut mittels HPLC analysiert.

In Vial 1 wurde keine Veränderung der Psicose-Menge nach Zusatz der Epimerase festgestellt, was für L-Psicose als Produkt spricht.

In Vial 2 wurde eine Mischung von 25:75 (Psicose:Fructose) erhalten, was sich sehr gut mit den beobachteten Gleichgewichtsverhältnissen der Epimerisierung aus der Literatur deckt (Zhang et al., 2016; Jiang et al., 2020). Somit handelt es sich beim Produkt um D-Psicose.

Des Weiteren konnte in diesem Beispiel gezeigt werden, dass neben der NAD-abhängigen Oxidoreduktase (Xylitol-Dehydrogenase) aus dem Beispiel 1 auch eine NADP-abhängige Oxidoreduktase (Short-Chain-Dehydrogenase/Reduktase) für die Oxidation von Allitol zu D-Psicose verwendet werden kann. Das letztgenannte Enzym katalysiert auch die Reduktion von D-Psicose zu Allitol.

### Literatur

Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Hough, L., & Stacey, B. E. (1996). Variation in the allitol content of Itea plants during photosynthesis. Phytochemistry, 5(1), 171-175. https://doi.org/10.1016/S0031-9422(00)85095-5
Oshima, H., Kimura, I., & Izumori, K. (2006). Psicose Contents in Various Food Products and its Origin. Food Science and Technology Research, 12(2), 137-143. https://doi.org/10.3136/fstr.12.137
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Ahmed, A., Khan, T. A., Ramdath, D. D., Kendall, C. W. C., & Sievenpiper, J. L. (2022). Rare sugars and their health effects in humans: a systematic review and narrative synthesis of the evidence from human trials, Nutrition Reviews, 80(2), 255-270. https://doi.org/10.1093/nutrit/nuab012
Chen, Z., Gao, X.-D., & Li, Z. (2022). Recent Advances Regarding the Physiological Functions and Biosynthesis of D-Allulose. Frontiers in Microbiology, 13, 881037. https://doi.org/10.3389/fmicb.2022.881037
Doner, L. W. (1979). Isomerization of D-fructose by base: Liquid-chromatographic evaluation and the isolation of D-psicose. Carbohydrate Research, 70(2), 209-216. https://doi.org/10.1016/S0008-6215(00)87101-3
Bílik, V., & Tihlärik, K. (1974). Reactions of saccharides catalyzed by molybdate ions. IX. Epimerization of ketohexoses. Chemical Papers, 28(1), 106-109. https://www.chemicalpapers.com/?id=7&paper=5533
Izumori, K., Khan, A. R., Okaya, H., & Tsumura, T. (1993). A New Enzyme, D-Ketohexose 3-Epimerase, from Pseudomonas sp. ST-24. Bioscience, Biotechnology, and Biochemistry, 57(6), 1037-1039. https://doi.org/10.1271/bbb.57.1037
Kim, N.-H., Kim, H.-J., Kang, D.-I., Jeong, K.-W., Lee, J.-K., Kim, Y., & Oh, D.-K. (2008). Conversion Shift of D-Fructose to D-Psicose for Enzyme-Catalyzed Epimerization by Addition of Borate. Applied and Environmental Microbiology, 74(10), 3008-3013. https://doi.org/10.1128/AEM.00249-08
Lim, B.-C., Kim, H.-J., & Oh, D.-K. (2009). A stable immobilized D-psicose 3-epimerase for the production of D-psicose in the presence of borate. Process Biochemistry, 44(8), 822-828. https://doi.org/10.1016/j.procbio.2009.03.017
Zhu, P., Zeng, Y., Chen, P., Men, Y., Yang, J., Yue, X., Zhang, J., Zhu, Y., & Sun, Y. (2020). A one-pot twoenzyme system on the production of high value-added D-allulose from Jerusalem artichoke tubers. Process Biochemistry, 88, 90-96. https://doi.org/10.1016/j.procbio.2019.10.006
Li, C. Li, L., Feng, Z., Guan, L., Lu, F., & Qin, H.-M. (2021). Two-step biosynthesis of D-allulose via a multienzyme cascade for the bioconversion of fruit juices. Food Chemistry, 357, 129746. https://doi.org/10.1016/j.foodchem.2021.129746
Juneja, A., Zhang, G., Jin, Y.-S., & Singh, V. (2019). Bioprocessing and technoeconomic feasibility analysis of simultaneous production of d-psicose and ethanol using engineered yeast strain KAM-2GD. Bioresource Technology, 275, 27-34. https://doi.org/10.1016/j.biortech.2018.12.025
Patel, S. N., Singh, V., Sharma, M., Sangwan, R. S., Singhal, N. K., & Singh, S. P. (2018). Development of a thermo-stable and recyclable magnetic nanobiocatalyst for bioprocessing of fruit processing residues and D-allulose synthesis. Bioresource Technology, 247, 633-639. https://doi.org/10.1016/j.biortech.2017.09.112
Yang, P., Zhu, X., Zheng, Z., Mu, D., Jiang, S., Luo, S., Wu, Y., & Du, M. (2018). Cell regeneration and cyclic catalysis of engineered Kluyveromyces marxianus of a D-psicose-3-epimerase gene from Agrobacterium tumefaciens for D-allulose production. World Journal of Microbiology and Biotechnology, 34, 65. https://doi.org/10.1007/s11274-018-2451-6
Dedania, S. R., Patel, V. R., Soni, S. S., & Patel, D. H. (2020). Immobilization of Agrobacterium tumefaciens D-psicose 3-epimerase onto titanium dioxide for bioconversion of rare sugar. Enzyme and Microbial Technology, 140, 109605. https://doi.org/10.1016/j.enzmictec.2020.109605
Li, Y., Shi, T., Han, P., & You, C. (2021). Thermodynamics-Driven Production of Value-Added D-Allulose from Inexpensive Starch by an In Vitro EnzymaticSynthetic Biosystem. ACS Catalysis, 11(9), 5088-5099. https://doi.org/10.1021/acscatal.0c05718
Wang, W., Yang, J., Sun, Y., Li, Z., & You, C. (2020). Artificial ATP-Free in Vitro Synthetic Enzymatic Biosystems Facilitate Aldolase-Mediated C-C Bond Formation for Biomanufacturing. ACS Catalysis, 10(2), 1264-1271. https://doi.org/10.1021/acscatal.9b04696
Li, Z., Li, F., Cai, L., Chen, Z., Qin, L., & Gao, X.-D. (2020). One-Pot Multienzyme Synthesis of Rare Ketoses from Glycerol. Journal of Agricultural and Food Chemistry, 68(5), 1347-1353. https://doi.org/10.1021/acs.jafc.9b06748
Xiao, Q., Niu, J., Liu, H., Liu, Y., & Zhou, X. (2019). High Conversion of D-Fructose into D-Allulose by Enzymes Coupling with an ATP Regeneration System. Molecular Biotechnology, 61, 432-441. https://doi.org/10.1007/s12033-019-00174-6
Zhang, J., Luo, W., Wang, Z., Chen, X., Lv, P., & Xu, J. (2021). A novel strategy for D-psicose and lipase co-production using a co-culture system of engineered Bacillus subtilis and Escherichia coli and bioprocess analysis using metabolomics. Bioresources and Bioprocessing, 8, 77. https://doi.org/10.1186/s40643-021-00429-8
Itoh, H. & Izumori, K. (1996). Enzymatic production of L-tagatose and L-fructose from L-sorbose and L-psicose, respectively. Journal of Fermentation and Bioengineering, 81(4), 351-353. https://doi.org/10.1016/0922-338X(96)80590-3
Rao, D., Gullapalli, P., Yoshihara, A., Jenkinson, S. F., Morimoto, K., Takata, G., Akimitsu, K., Tajima, S., Fleet, G. W. J., & Izumori, K. (2008). Direct Production of L-Tagatose from L-Psicose by Enterobacter aerogenes 230S. Journal of Bioscience and Bioengineering, 106(5), 473-480. https://doi.org/10.1263/jbb.106.473
Sasahara, H., & Izumori, K. (2005). Production of L-talitol from L-psicose by Metschnikowia koreensis LA1 isolated from soy sauce mash. Journal of Bioscience and Bioengineering, 100(3), 335-338. https://doi.org/10.1263/jbb.100.335
Muniruzzaman, S., Mclntosh, M., Hossain, A., Izumori, K., & Bhattacharjee, P. S. (2016). A novel rare sugar inhibitor of murine herpes simplex keratitis. Journal of Pharmacological Sciences, 131(2), 126-130. https://doi.org/10.1016/j.jphs.2016.05.004
Yang, J., Li, J., Men, Y., Zhu, Y., Zhang, Y., Sun, Y., & Ma, Y. (2015). Biosynthesis of L-Sorbose and L-Psicose Based on C-C Bond Formation Catalyzed by Aldolases in an Engineered Corynebacterium glutamicum Strain. Applied and Environmental Microbiology, 81(13), 4284-4294. https://doi.org/10.1128/AEM.00208-15
Yang, J., Zhu, Y., Men, Y., Sun, S., Zeng, Y., Zhang, Y., Sun, Y. &, Ma, Y. (2016). Pathway Construction in Corynebacterium glutamicum and Strain Engineering To Produce Rare Sugars from Glycerol. Journal of Agricultural and Food Chemistry, 64(50), 9497-9505. https://doi.org/10.1021/acs.jafc.6b03423
Wen, L., Huang, K., Wei, M., Meisner, J., Liu, Y., Garner, K., Zang, L., Wang, X., Li, X., Fang, J., Zhang, H. & Wang, P. G. (2015). Facile Enzymatic Synthesis of Ketoses. Angewandte Chemie International Edition, 54(43), 12654-12658. https://doi.org/10.1002/anie.201505714
Lorillière, M., Dumoulin, R., L'enfant, M., Rambourdin, A., Thery, V., Nauton, L., Fessner, W.-D., Charmantray, F., & Hecquet, L. (2019). Evolved Thermostable Transketolase for Stereoselective Two-Carbon Elongation of Non-Phosphorylated Aldoses to Naturally Rare Ketoses. ACS Catalysis, 9(6), 4754-4763. https://doi.org/10.1021/acscatal.9b01339
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Gullapalli, P., Takata, G., Poonperm, W., Rao, D., Morimoto, K., Akimitsu, K., Tajima, S., & Izumori, K. (2007). Bioproduction of D-psicose from allitol with Enterobacter aerogenes IK7: a new frontier in rare ketose production. Bioscience, Biotechnology, and Biochemistry, 71(12), 3048-3054. https://doi.org/10.1271/bbb.70450
Poonperm, W., Takata, G., Ando, Y., Sahachaisaree, V., Lumyong, P., Lumyong, S., & Izumori, K. (2007). Efficient conversion of allitol to D-psicose by Bacillus pallidus Y25. Journal of Bioscience and Bioengineering, 103(3), 282-285. https://doi.org/10.1263/jbb.103.282
Izumori, K. (2006). Izumoring: a strategy for bioproduction of all hexoses. Journal of Biotechnology, 124(4), 717-722. https://doi.org/10.1016/j.jbiotec.2006.04.016
Hassanin, H. A. M., Mu, W., Koko, M. Y. F., Zhang, T., Masamba, K., Jiang, B. (2017). Allitol: production, properties and applications. International Journal of Food Science and Technology, 52(1), 91-97. https://doi.org/10.1111/ijfs.13290
Takeshita, K., Shimonishi, T., & Izumori, K. (1996). Production of L-psicose from allitol by Gluconobacter frateurii IFO 3254. Journal of Fermentation and Bioengineering, 81(3), 212-215. https://doi.org/10.1016/0922-338X(96)82210-0
Lin, B., & Tao, Y. (2017). Whole-cell biocatalysts by design. Microbial Cell Factories, 16, 106. https://doi.org/10.1186/s12934-017-0724-7
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Protein [Internet]. Bethesda (MD): National Library of Medicine (US), National Center for Biotechnology Information. Accession No. WP_063903495.1, SDR family oxidoreductase [Gluconobacter frateurii]. Verfügbar unter: https://www.ncbi.nlm.nih.gov/protein/WP_063903495.1/ (Zugriff am 14.03.2023)
Habenicht, A., Motejadded, H., Kiess, M., Wegerer, A., & Mattes, R. (1999). Xylose Utilisation: Cloning and Characterisation of the Xylitol Dehydrogenase from Galactocandida mastotermitis. Biological Chemistry, 380(12), 1405-1411. https://doi.org/10.1515/BC.1999.180
V. L. Louis, L. Despons, A. Friedrich, et al. (2012). Pichia sorbitophila, an Interspecies Yeast Hybrid, Reveals Early Steps of Genome Resolution After Polyploidization. G3 Genes/Genomes/Genetics, 2(2), 299-311. https://doi.org/10.1534/g3.111.000745
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39
Petschacher, B., Staunig, N., Müller, M., Schürrmann, M., Mink, D., De Wildeman, S., Gruber, K., & Glieder, A. (2014). COFACTOR SPECIFICITY ENGINEERING OF STREPTOCOCCUS MUTANS NADH OXIDASE 2 FOR NAD(P)+ REGENERATION IN BIOCATALYTIC OXIDATIONS. Computational and Structural Biotechnology Journal, 9(14), e201402005. https://doi.org/10.5936/csbj.201402005

## Patentansprüche

1. Verfahren zur Herstellung einer wässerigen Lösung enthaltend D-Psicose oder L-Psicose, indem aus D-Fructose, welche in einer wässerigen Lösung gelöst vorliegt, durch Behandeln mit einer Epimerase *in vitro* eine erste D-Psicose gebildet wird, wonach die erste D-Psicose durch Behandeln mit einer NAD(P)H-abhängigen Oxidoreduktase *in vitro* zu Allitol reduziert und nach Deaktivierung der Epimerase zur Bildung von D-Psicose bzw. von L-Psicose mit einer entsprechenden Oxidoreduktase behandelt wird, wonach die deaktivierte Epimerase und die Oxidoreduktasen entfernt werden.

2. Verfahren nach Anspruch 1, **dadurch gekennzeichnet, dass** der durch die Reduktion entstandene oxidierte Kofaktor NAD(P)⁺ mittels einer Alkoholdehydrogenase und einem sekundären Alkohol unter Bildung eines Ketons reduziert wird.

3. Verfahren nach Anspruch 2, **dadurch gekennzeichnet, dass** der sekundäre Alkohol 2-Propanol ist.

4. Verfahren nach einem der Ansprüche 1 bis 3, **dadurch gekennzeichnet, dass** es als Eintopf-Reaktion ohne Isolierung von Zwischenprodukten durchgeführt wird.

5. Verfahren nach einem der Ansprüche 1 bis 4, **dadurch gekennzeichnet, dass** die Enzyme als Lysat der entsprechenden, sie bildenden Zellen vorliegen.

6. Verwendung der Short-Chain-Dehydrogenase/Reduktase (SDR), erhältlich aus dem Spenderorganismus *Gluconobacter frateurii* (DSM 7146), zur Reduktion von D-Psicose zu Allitol.

7. Verwendung der Xylitol-Dehydrogenase (XDH, EC 1.1.1.9), erhältlich aus dem Spenderorganismus *Galactocandida mastotermitis* (*Candida* sp. HA167) zur Oxidation von Allitol zu D-Psicose.

8. Verwendung der Mannitol-Dehydrogenase-artigen Short-Chain-Dehydrogenase/Reduktase, erhältlich aus *Millerozyma farinosa* (*Pichia sorbitophila*) CBS 7064, zur Oxidation von Allitol zu L-Psicose.
